# EUROPEAN PATENT SPECIFICATION

(11) **EP 2 757 150 B1**
(45) Date of publication and mention of the grant of the patent: **17.01.2018**
(21) Application number: 12832197.3
(22) Date of filing: 11.09.2012
(51) Int. Cl.: C12N 1/20, A23L 29/00, A23L 11/00, C12R 1/125

(54) **NEW BACILLUS SUBTILIS SUBSP. NATTO AND NATTO PRODUCED USING SAME**
NEUER BACILLUS SUBTILIS SUBSP. NATTO UND HERSTELLUNG VON NATTO DAMIT
NOUVEAU BACILLUS SUBTILIS SSP. NATTO ET NATTO PRODUIT L'UTILISANT

(30) Priority: 13.09.2011 JP 2011198971
(43) Date of publication of application: 23.07.2014
(73) Proprietor: Azuma Foods Co., Ltd., Utsunomiya-shi, Tochigi 3291115 (JP)
(72) Inventor: OKUHATA, Noriei, Utsunomiya-shi Tochigi 329-1115 (JP); USHIKU, Yoshihiro, Utsunomiya-shi Tochigi 329-1115 (JP); HAYASHI, Yoshiko, Utsunomiya-shi Tochigi 329-1115 (JP); YOSHIHARA, Keiko, Utsunomiya-shi Tochigi 329-1115 (JP); WATANABE, Sugio, Oyama-shi Tochigi 323-0807 (JP)
(74) Representative: Hoffmann Eitle
(86) International application number: PCT/JP2012/073175
(87) International publication number: WO 2013/039058

(56) References cited:
- JP-A- S6 455 156
- JP-A- H06 269 281
- JP-A- 2006 025 785
- JP-A- 2006 067 992
- JP-A- 2007 143 467
- JP-A- 2007 209 294
- SAORI TAKAHASHI ET AL: "Soft Natto Manufactured with Starter of Bacillus subtilis KFP 843 Isolated from Douchi", NIPPON SHOKUHIN KAGAKU KOGAKU KAISHI, vol. 52, no. 10, 1 January 2005 (2005-01-01), pages 454-461, XP55185080, ISSN: 1341-027X, DOI: 10.3136/nskkk.52.454
- SAORI MITSUBOSHI ET AL: "Development of Soft, Sticky Itohiki-natto Manufactured for Senior Citizens", NIPPON SHOKUHIN KAGAKU KOGAKU KAISHI, vol. 53, no. 9, 1 January 2006 (2006-01-01), pages 466-473, XP55185075, ISSN: 1341-027X, DOI: 10.3136/nskkk.53.466
- JOMKHWAN MEERAK ET AL: "Phylogeny of [gamma]-polyglutamic acid-producing Bacillus strains isolated from fermented soybean foods manufactured in Asian countries", THE JOURNAL OF GENERAL AND APPLIED MICROBIOLOGY, vol. 53, no. 6, 1 December 2007 (2007-12-01), pages 315-323, XP55185133, ISSN: 0022-1260, DOI: 10.2323/jgam.53.315

### [Sequence Listing]

### SEQUENCE LISTING

<110> Azuma foods co., ltd.
<120> New Bacillus subtilis and Bacillus Natto produced by using the same
<130> P-613
<140> EP12832197.3
   <141> 2012-09-11
<150> JP2011-198971
   <151> 2011-09-13
<160> 1
<170> PatentIn version 3.5
<210> 1
   <211> 1475
   <212> DNA
   <213> Bacillus subtilis
<400> 1
   gacgaacgct ggcggcgtgc ctaatacatg caagtcgagc ggacagatgg gagcttgctc      60
   cctgatgtta gcggcggacg ggtgagtaac acgtgggtaa cctgcctgta agactgggat     120
   aactccggga aaccggggct aataccggat ggttgtttga accgcatggt tcaaacataa     180
   aaggtggctt cggctaccac ttacagatgg acccgcggcg cattagctag ttggtgaggt     240
   aacggctcac caaggcaacg atgcgtagcc gacctgagag ggtgatcggc cacactggga     300
   ctgagacacg gcccagactc ctacgggagg cagcagtagg gaatcttccg caatggacga     360
   aagtctgacg gagcaacgcc gcgtgagtga tgaaggtttt cggatcgtaa agctctgttg     420
   ttagggaaga acaagtaccg ttcgaatagg gcggtacctt gacggtacct aaccagaaag     480
   ccacggctaa ctacgtgcca gcagccgcgg taatacgtag gtggcaagcg ttgtccggaa     540
   ttattgggcg taaagggctc gcaggcggtt tcttaagtct gatgtgaaag cccccggctc     600
   aaccggggag ggtcattgga aactggggaa cttgagtgca gaagaggaga gtggaattcc     660
   acgtgtagcg gtgaaatgcg tagagatgtg gaggaacacc agtggcgaag gcgactctct     720
   ggtctgtaac tgacgctgag gagcgaaagc gtggggagcg aacaggatta gataccctgg     780
   tagtccacgc cgtaaacgat gagtgctaag tgttaggggg tttccgcccc ttagtgctgc     840
   agctaacgca ttaagcactc cgcctgggga gtacggtcgc aagactgaaa ctcaaaggaa     900
   ttgacggggg cccgcacaag cggtggagca tgtggtttaa ttcgaagcaa cgcgaagaac     960
   cttaccaggt cttgacatcc tctgacaatc ctagagatag gacgtcccct tcgggggcag    1020
   agtgacaggt ggtgcatggt tgtcgtcagc tcgtgtcgtg agatgttggg ttaagtcccg    1080
   caacgagcgc aacccttgat cttagttgcc agcattcagt tgggcactct aaggtgactg    1140
   ccggtgacaa accggaggaa ggtggggatg acgtcaaatc atcatgcccc ttatgacctg    1200
   ggctacacac gtgctacaat ggacagaaca aagggcagcg aaaccgcgag gttaagccaa    1260
   tcccacaaat ctgttctcag ttcggatcgc agtctgcaac tcgactgcgt gaagctggaa    1320
   tcgctagtaa tcgcggatca gcatgccgcg gtgaatacgt tcccgggcct tgtacacacc    1380
   gcccgtcaca ccacgagagt ttgtaacacc cgaagtcggt gaggtaacct tttaggagcc    1440
   agccgccgaa ggtgggacag atgattgggg tgaag                               1475

## Description

### TECHNICAL FIELD

The present invention relates to a strain separated from nature and natto (fermented soybeans) produced by using the strain.

### BACKGROUND ART

As is known in the related art, natto is produced in such a manner that Bacillus subtilis var. natto is inoculated on steam-cooked soybeans, followed by fermentation and aging in a fermentation chamber. From the viewpoints of the effect of regulating intestines of the human body and a thrombolytic effect of Bacillus subtilis var. natto, eating natto is encouraged. This, natto is provided as a so-called health food.

Bacillus subtilis var. natto is a kind of Bacillus subtilis and is abound in rice straws. For example, according to Natto Encyclopedia published by Japan natto cooperative society federation, it is pointed out that ten millions of Bacillus subtilis var. natto are attached to one strand of rice straws produced in Japan in a spore state.

As described above, Bacillus subtilis var. natto inhabits in the natural world. Bacteria having different attributes can be separated from a large variety and a large number of Bacillus subtilis var. natto. Accordingly, it is possible to obtain natto different from ordinary natto.

In general, natto contains a large number of viscous substances that have sticky and slimy feelings, which are inherent characteristics of natto. Further, natto has inherent odor of natto. According to these, natto is a food which is liked by a person and disliked by other person.

### LIST OF PRIOR ART DOCUMENTS

### NON-PATENT DOCUMENTS

"
Chiiki Shigen Katsuyou Shokuhin Kakou Souran Vol. 5 (Translation: Local Resource Utilization, Comprehensive Food Processing, Vol. 5" (Sugio Watanabe, et al., published by Rural Culture Association Japan)
"Shokuhin Kakou Series (5) Natto (Translation: Food Processing Series (5) Natto)" (Sugio Watanabe, published by Rural Culture Association Japan)
"Industrialization of Indigenous Fermented Foods" (Sugio Watanabe, et al., published by MERCEL DEKKER INC,JAPAN)
"Hakkou To Zyouzou <3> (Translation: Fermentation and Brewage <3>)" (Sugio Watanabe, et al., published by Kourin,Japan)
"Tsukutte Asobou (2) Natto No Ehon (Translation: Let's Make and Play (2), Picture Book of Natto)" (Sugio Watanabe, et al., published by Rural Culture Association Japan)
"Natto No Kagaku - Saishin Zyouhouniyouru Sougouteki Kousatsu (Translation: Science of Natto - Comprehensive Study Based on the Latest Information)" (Sugio Watanabe, et al., published by KENPAKUSHA, JAPAN)
"Natto No Subete (Translation: All about Natto)" (Sugio Watanabe, et al., published by SCIENCE FORUM, JAPAN)
"Natto No Kenkyuhou (Translation: Research Methodology of Natto)" (edited by Sugio Watanabe, et al., published by Kouseisha-kouseikaku Corporation, JAPAN)
Shokuhinchishiki Mini Book Series Natto Nyuumon (Translation: Food Knowledge, Mini Book Series: Introduction to Natto)" (Sugio Watanabe, published by JAPAN FOOD JOURNAL CO., LTD.)
"Natto No Seizougizyutsu To Housoukoutei (Translation: Production Technology and Packing Process of Natto)" (1985-10 Food Science, published by Shokuhin To Kagakusha, JAPAN)
"Daizukougyou - Sono Hatten No Katei To Genzyou (Translation: Soybean Industry -Its Development Process and Current Status)" (1991-2/3 combined number, published by Institute of soybean stability association, JAPAN)
"Natto Seizou Gizyutsu No Kadai · Kokusai Gizyutsu Iten (Translation: Problems of Natto Production Technology and International Technology Transfer)" (1994-10 Natto to Gizyutsu (Translation: Natto and Technology) published by Food Journal Co., Ltd. JAPAN)
"Microcalorimetric Analysis of Fermentation of Natto, a Traditional Japanese Food" (1999-05-15, published by The Japan Society for Food Science and Technology)
"Seicho Shizyou: Natto Seisan No Kougyouka To Kokusaika (Translation: Growing Market: Industrialization and Internationalization of Natto Production)" (2000-09-01, Journal of Technology Research for Agriculture, Forestry and Fisheries, JAPAN)
"Development of Low-Odor Natto produced with Leucine-requiring Mutants of Elastase-Producing Natto Bacillus" (2001-04-15, Journal of The Japan Society for Food Science and Technology)
"Itohiki Natto Seizouhou No Kairyo (Translation: Improvement of Producing Method of Natto having sticky threads)" (2001-04-15, Journal of The Japan Society for Food Science and Technology)
"Spore Formation in Natto Bacilli under Environmental Stress " (2006-03-15, Journal of The Japan Society for Food Science and Technology)
"Development of Soft, Natto having less sticky threads produced for Aged persons" (2006-09, Journal of The Japan Society for Food Science and Technology)
"Takabashikin Kara Bunrishita Nattokin KFP No Shokuhin Ekisuniyoru Houshikeisei (Translation: Spore Formation in Bacillus subtilis var. natto KFP Separated from Takabashi Bacteria by Food Extract)" (2007-09, Journal of The Japan Society for Food Science and Technology)

### PATENT DOCUMENTS

Patent Document 1: Japanese Patent Publication No. 2554454 B1
Patent Document 2: Japanese Patent Publication No. 2006-067992 A
Patent Document 3: Japanese Patent Publication No. 2006-314252 A

### DISCLOSURE OF THE INVENTION

### PROBLEMS TO BE SOLVED BY THE INVENTION

As described above, since natto has viscosity and natto odor, natto may be kept away from eating it in some cases. In view of this circumstance, viscosity and natto odor are tried to be reduced and natto is required to have an improved image as a popular food.

The inventors of the present invention have started the study on Bacillus subtilis var. natto since 1980. The inventors collected soils and rice straws from traditionally famous natto production regions thonghout the entire state of Japan and performed separation of Bacillus subtilis var. natto from the collected soils and rice straws and collection thereof . As a result of continuing a property investigation, the inventors reached a conclusion that more than one thousand and hundreds kinds of Bacillus subtilis var. natto had been inhabited in the collected and owned soils and rice straws.

Accordingly, an object of the present invention is to provide a strain capable of producing natto having reduced viscosity and natto odor by making a selection from Bacillus subtilis var. natto obtained by the inventor of the present invention, and provide natto produced by using the same.

### MEANS FOR SOLVING PROBLEMS

The inventors of the present invention had produced natto using Bacillus subtilis var. natto collected from the rice straws obtained and owned by the invention. Then, the invention was able to obtain natto having a small number of viscous substances that have sticky and slimy feelings, which are inherent characteristics of natto, and further having little natto odor, which is an inherent characteristic of natto. Consequently, the inventors considered this as new Bacillus subtilis var. natto and then named this "AZ-5512". Then, 16S rDNA base sequence analysis and physiological/biochemical property tests were carried out on "AZ-5512". From the results of the analysis and the tests, "AZ-5512" was identified as bacteria belonging to Bacillus subtilis and was deposited with National Institute of Advanced Industrial Science and Technology · International Patent Organism Depositary (Deposit Number: FERM P-22135).

### EFFECTS OF THE INVENTION

According to the present invention, it is possible to obtain natto having reduced viscosity and natto odor. In addition, natto having a lot of sweetness is obtained from Bacillus subtilis var. natto of the present invention and it is found that Bacillus subtilis var. natto of the present invention has a characteristic to make natto soft, or the like.

### BRIEF DESCRIPTION OF DRAWINGS

Fig. 1 is a table presenting a homologous rate of the present strain AZ-5512 (SIID10004) with B. subtilis subsp. inaquosorum and B. tequilensis;
Fig. 2 is a diagram illustrating base sequence comparisons of the present strain AZ-5512 (SIID10004) with B. subtilis subsp. and subtilis;
Fig. 3 is a diagram illustrating base sequence comparisons of the present strain AZ-5512 (SIID10004) with B. subtilis subsp. and subtilis (continued from Fig. 2);
Fig. 4 is a diagram illustrating base sequence comparisons of the present strain AZ-5512 (SIID10004) with B. subtilis subsp. and subtilis (continued from Fig. 3);
Fig. 5 is a table presenting a result of a first phase test;
Fig. 6 is a table presenting a result of a second phase test (AP150CHB);
Fig. 7 is a table relating to the explanation of AP150CHB item in Fig. 6;
Fig. 8 is a table presenting a result of the second phase test (additional experiment);
Fig. 9 is a table presenting a sensory test result of natto produced by using the present strain (AZ-5512) and of natto currently on the market.
Fig. 10 is a diagram illustrating nutrient components of natto produced by using the present strain (AZ-5512);
Fig. 11 is a diagram illustrating free amino acid contained in 100 g of natto produced by using the present strain (AZ-5512);
Fig. 12 is a comparative table of a viscosity of natto produced by using the present strain (AZ-5512) with that of natto fermented using Miyagino bacteria;
Fig. 13 is a table presenting volatile components of natto produced by using the present strain (AZ-5512) and of natto currently on the market;
Fig. 14 is a table presenting hardness and reduction rates of hardness of steam-cooked soybeans and of natto produced by using the present strain (AZ-5512);
Fig. 15 is a table presenting the comparison test result of softening degrees of natto produced from heterogeneous soybeans by using a commercially available strain and natto produced from heterogeneous soybeans by using the present strain (AZ-5512);
Fig. 16 is a table presenting the comparison test result of softening degrees of natto produced from heterogeneous soybeans by using a commercially available strain and natto produced from heterogeneous soybeans by using the present strain (AZ-5512); and
Fig. 17 is a table presenting the comparison test result of softening degrees of natto produced from heterogeneous soybeans by using a commercially available strain and natto produced from heterogeneous soybeans by using the present strain (AZ-5512).

### BEST MODE S FOR CARRYING OUT THE INVENTION

### Regarding identification of the present strain (AZ-5512)

The applicant of the present application requested 16S rDNA base sequence analysis and physiological/biochemical property tests on the present strain to Techno Suruga Laboratory Co., Ltd. located at 330 Nagasaki, Shimizu-ku, Shizuoka-shi, Shizuoka-ken, Japan (hereinafter, simply referred to as "Techno Suruga Laboratory Co., Ltd.") so as to acquire the following test results. Note that, in drawings and tables, the accession number of the present strain (AZ-5512), that is a specimen, at Techno Suruga Laboratory Co., Ltd. is registered as a registration No. "SIID10004". The specimen delivery from the inventor of the present invention to Techno Suruga Laboratory Co., Ltd. was made on April 7, 2011, the source for separation was soil, and the test result was reported on May 30, 2011.

### Object

Estimation of the belonging taxon of specimen is carried out from results of 16S rDNA (16S rRNA gene) base sequence analysis, morphology observation and physiological/biochemical property tests (hereinafter, referred to as "a first phase test for bacteria" and "a second phase test for bacteria").

### Method

### 1. Culture Condition

Strains, which were cultured under the following condition, are used as specimens of bacterial bodies.
- Culture medium Nutrient agar (Oxoid, Hampshire, England)
- Culture temperature 30°C
- Culture period 24 hours

### 2. 16S rDNA-Full

Operations from extraction to cycle sequence were performed on the basis of each protocol.
- DNA extraction InstaGene Matrix (BIO RAD, CA, USA)
- PCR PrimeSTAR HS DNA Polymerase (TAKARA BIO INC., Shiga, Japan)
- Cycle sequence BigDye Tenllinator v3.1 Cycle Sequencing Kit (Applied Biosystems, CA, USA)
- Used primer PCR amplification: 9F, 1510R sequence: 9F785F, 802R, 1510R
- Sequence ABI PRISM 3130 xl Genetic Analyzer System (Applied Biosystems, CA, USA)
- Sequence determination ChromasPro 1.4 (Technelysium Pty Ltd., Tewantin, AUS)
- Homology search and simplified molecular phylogenetic analysis Software; Apollon 2.0 (Techno Suruga Laboratory Co., Ltd., Shizuoka, Japan) Database; Apollon DB-BA 6.0 (Techno Suruga Laboratory Co., Ltd., Shizuoka, Japan) International Nucleotide Sequence Database (GenBank/DDBJ/EMBL)

### 3. First Phase Test of Bacteria

Catalase reaction, oxidase reaction, production of acid/gas from glucose and oxidation/fermentation (O/F) of glucose were tested on the basis of morphology observation by using an optical microscope and methods of BARROW et al.
- Gram staining Faber G "NISSUI" (NISSUI PHARMACEUTICAL CO., LTD., Tokyo, Japan)
- Microscope Optical microscope BX50F4 (Olympus, Tokyo, Japan)

### 4. Second Phase Test of Bacteria

The following kit was used in the second phase test of bacteria. Further, an additional test was carried out according to the agreement of technical cooperation with NCIMB Ltd., U.K. (http://www.ncimb.comuk/) and relevant literatures of classification and identification.
- Used kit API50CHB (bioMerieux, Lyon, France)

### Resuls

### 1. 16S rDNA (16S rRNA gene) Base Sequence Analysis

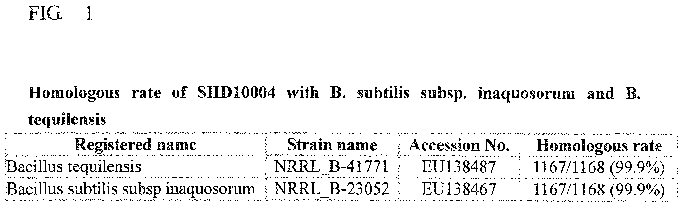

As the results of homology search with respect to Apollon DB-BA 6.0 using BLAST and homology search with respect to GenBank/DDBJ/EMBL, the 16S rDNA base sequence of AZ-5512 (SIID10004) exhibited high homology with 16S rDNA base sequence of the genus Bacillus. However, since the 16S rDNA base sequence derived from a type strain was not searched, two types of 16S rDNA base sequences were acquired and BLAST homology search was carried out. As a result, 16S rDNA base sequence of AZ-5512 (SIID10004) exhibited high homology, that is, 99.9% of homologous rate with respect to B. subtilis subsp. inaquosorum NRRL B-23052 strain and to B. tequilensis NRRL B-41771 strain, respectively (see Fig. 1).

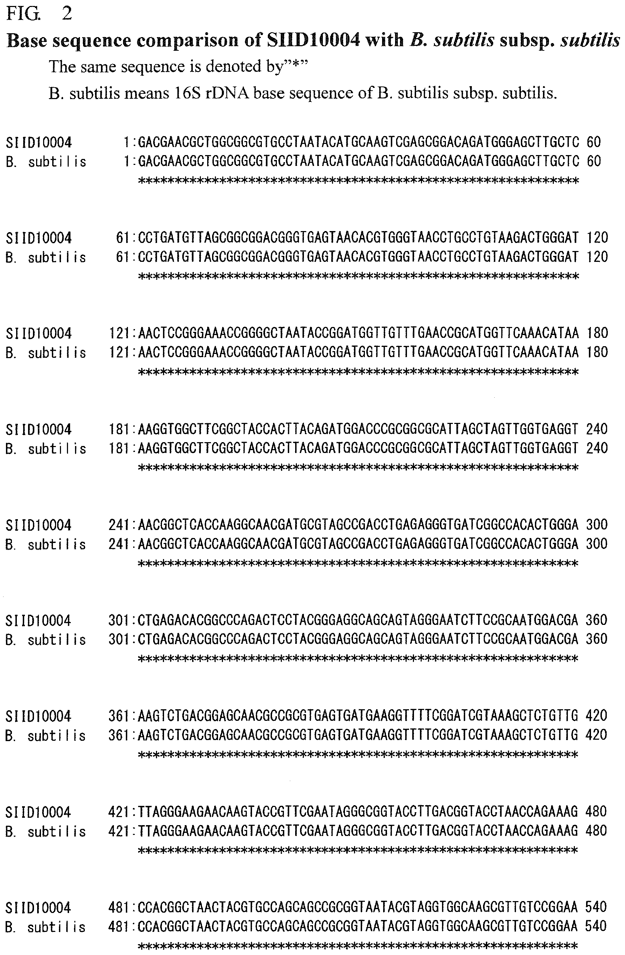

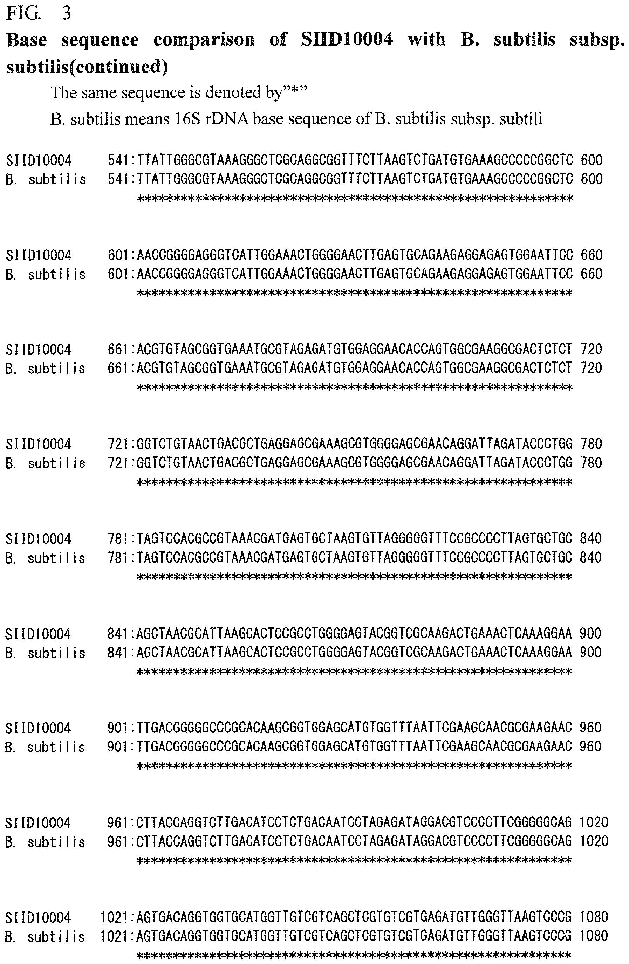

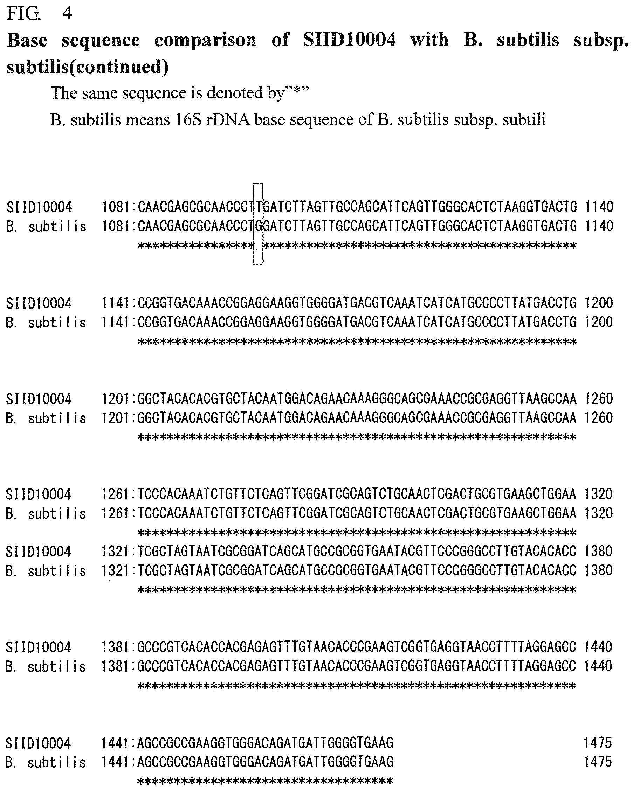

From the above description, it is considered that AZ-5512 (SIID10004), at a genus level, belongs to the genus Bacillus. Moreover, a single base difference between the 16S rDNA base sequences of AZ-5512 (SIID10004) and the B. subtilis subsp. subtilis was confirmed, and thus it was found that they were distinctly different from each other (see Figs. 2 to 4). As described above, from the result of the 16S rDNA base sequence analysis, it was assumed that AZ-5512 (SIID10004) was Bacillus sp. closely relating to B. subtilis. Note that, in "the diagram illustrating base sequence comparisons of the present strain AZ-5512 (SIID10004) with B. subtilis subsp. subtilis" of Figs. 2 to 4, the same sequence is denoted by "*" and B. subtilis means the 16S rDNA base sequence of B. subtilis subsp. subtilis.

### 2. Physiological/Biochemical Property Test

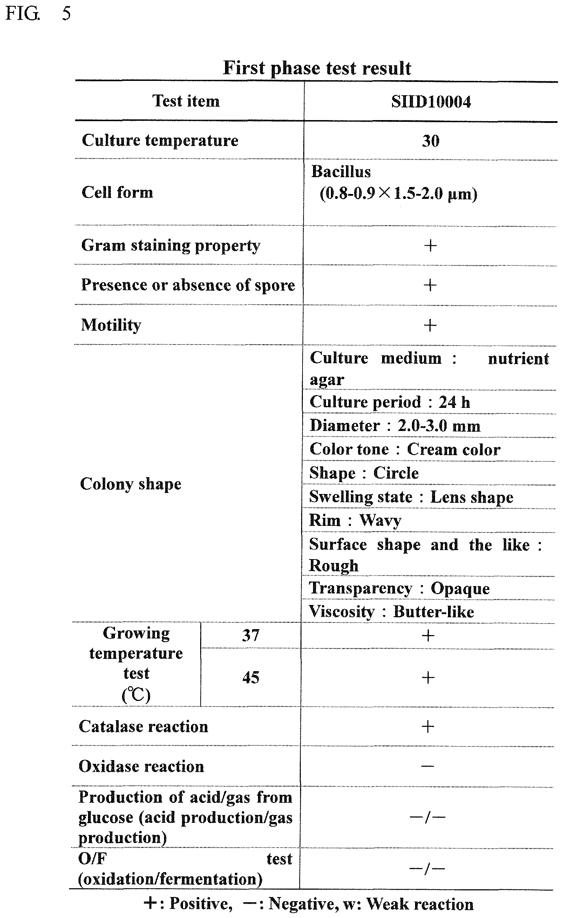

As the result of the first phase test of bacteria, AZ-5512 (SIID10004) was Gram-positive spore-bearing bacillus having motility, and the expansion of the bacterial body due to the spore was not recognized. The catalase reaction exhibited positive and the oxidase reaction exhibited negative (see Fig. 5). These properties are considered to be substantially coincident with properties of the genus Bacillus.

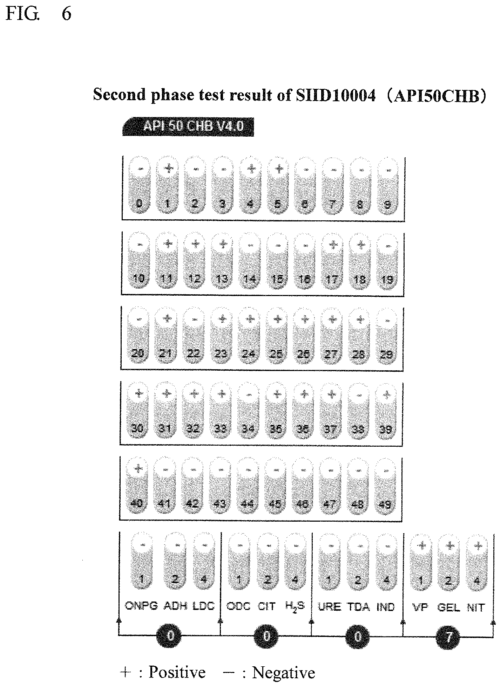

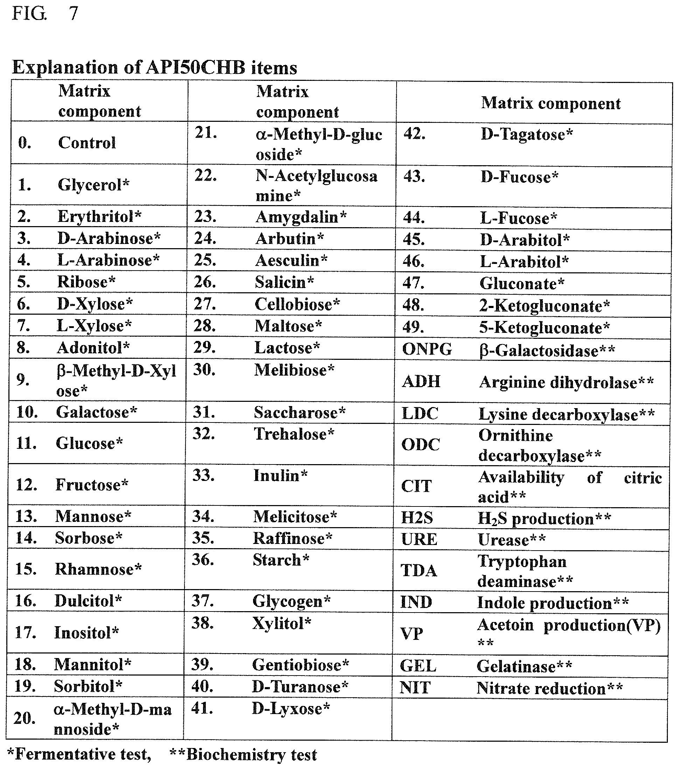

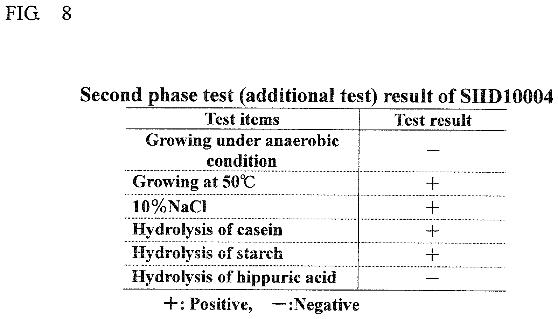

As the result of the second phase test of bacteria, AZ-5512 (SIID10004) oxidized glycerol, L-arabinose, ribose, glucose and the like but did not oxidize D-arabinose, D-xylose and the like, and produced acetoin, hydrolyzed gelatin, and reduced a nitrate (see Figs. 6 and 7). Moreover, AZ-5512 (SIID10004) did not grow under an anaerobic condition but grew at 50°C and 10% NaCl, and hydrolyzed casein and starch, but did not hydrolyze hippuric acid (see Fig. 8). These properties are considered to be substantially coincident with properties of B. subtilis that was suggested to have alliance from the result of 16S rDNA base sequence analysis.

As described above, in 16S rDNA base sequence analysis, slight difference between AZ-5512 (SIID10004) and B. subtilis subsp. subtilis was recognized but distinct difference between them was only one base. Further, the results of physiological/biochemical property tests of AZ-5512 (SIID10004) were substantially coincident with B. subtilis. According to this, the difference of 16S rDNA base sequence which was recognized between AZ-5512 (SIID10004) and B. subtilis subsp. subtilis can be grasped as an intraspecific difference in the B. subtilis.

On the other hand, estimation of the belonging taxon in B. subtilis at a subspecies level cannot be carried out in either the 16S rDNA base sequence analysis or in the physiological/biochemical property tests, therely the AZ-5512 (SIID10004) was estimated to be Bacillus subtilis from the result of the identification test at this time.

As described above, from the result obtained by identifying this strain on the basis of the acquired information relating to morphological, cultural, physiological properties and 16S rRNA gene, it was found that AZ-5512 was a bacterium belonging to Bacillus subtilis.

Note that, various kinds of bacterial isolates were inoculated on each breed of steam-cooked soybeans, the inoculated soybeans were filled in a container for natto, and then tests for production of natto by using the bacterial isolates were collectively performed in a small-sized automatic natto producing apparatus for laboratory. In addition, from the result of the sensory evaluation after production, if there are products which are considered to need to be subjected to a test, physical and chemical tests were further carried out thereon. Accordingly, superior natto-producing bacteria can be found so as to be used in industrial production, and thus unique natto product can be produced. The present strain (AZ-5512) was also selected by the above-described process.

The production of natto by using the present strain (AZ-5512) is carried out by the following process. In other words, typical soybeans as raw materials for natto were soaked in a typical and appropriate manner, and appropriately steamed at a temperature in a range of 120°C to 130°C for 15 minutes to 60 minutes. Then 1.0 × 10³ to 1.0 × 10⁵ spores of strains are inoculated on each 1 g of the steamed soybeans, the inoculated soybeans are filled in a container for fermentation under the condition of room temperature of 36°C to 40°C and in high humidity. The production of natto by using the present strain (AZ-5512) was also performed according to the above process.

In the fermentation process of the natto produced by using the present strain (AZ-5512), a desirable natto product can be obtained under the fermentation condition in which room temperature is adjusted to appropriately 37°C to 40°C, and after the product temperature reaches a target temperature of 50°C, the product temperature is gradually decreased by taking time.

After the production test of the natto, the following sensory evaluation was performed.

A method of sensory evaluation is as follows. Three natto products of the natto produced by using the present strain (AZ-5512), and products A and B produced on the same date and currently on the market were taken out from a refrigerator after two days of production, were left to stand at room temperature for one hour, and thereafter the sensory evaluation was carried out.

Appearance, color, and the like were examined on the upper surface and the lower surface. After agitating natto 20 times with disposable chopsticks, 1/2 to 1/3 of natto (in the case of 50 g natto) was picked up to a height of approximately 40 cm with the disposable chopsticks and then stickiness thereof was observed. Subsequently, flavor, hardness and taste of beans were examined.

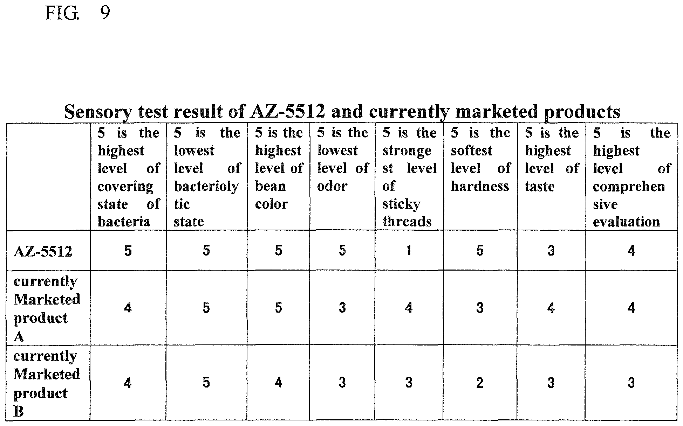

Five-grade evaluation was performed by five people who belong to Quality Management Section of the applicant of the present application. In other words, the natto products A and B currently on the market were simultaneously examined and the evaluation was performed on the grade of five scales: "3 (normal)", "5 (good)", and "1 (bad)" (see Fig. 9).

From the result of the above-described sensory evaluation of the natto, the following matters were found.

As for appearance, the moss of Bacillus subtilis var. natto was coated thickly and the natto had cream color, which is favorable. Accordingly, the natto produced by using the present strain (AZ-5512) was superior to the currently marketed natto products. As for natto odor, the natto produced by using the present strain (AZ-5512) had slightly sweet smell and no natto odor. As having no natto odor, the natto produced by using the present strain is considered to be a product sufficiently acceptable to people who dislike natto. As for softness, the natto produced by using the present strain (AZ-5512) is a very soft product to be eaten easily and has an easy processing property as a material for food processing. Although sticky threads are a characteristic of natto, it was confirmed that the natto produced by using the present strain (AZ-5512) had little sticky threads. Moreover, when foreigners were allowed to taste the natto produced by using the present strain (AZ-5512), it was found that foreigners, who dislike sticky and slimy texture of viscous substances, were able to eat the natto produced by using the present strain.

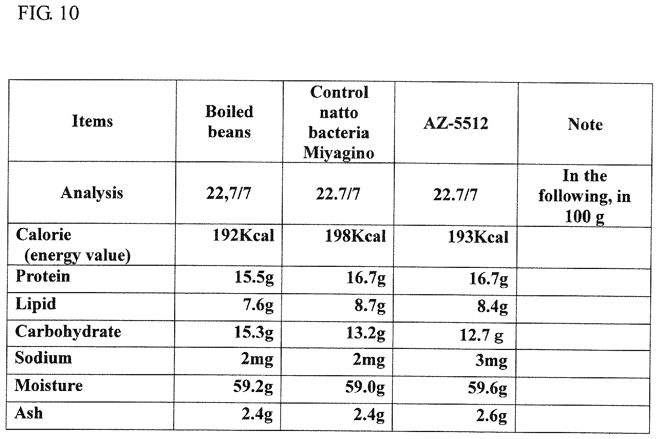

Next, component analysis of the natto produced by using the present strain (AZ-5512) and the natto produced by using the general Bacillus subtilis var. natto was carried out. The details are as presented in Fig. 10.

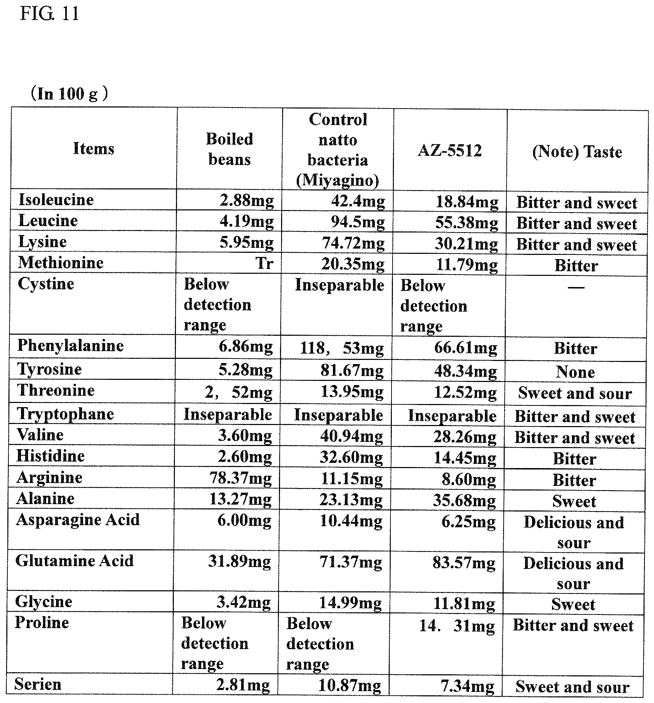

Furthermore, the free amino acid was compared between the natto produced by using the present strain (AZ-5512) and the natto produced by using the general Bacillus subtilis var. natto. According to the result of comparison, the natto produced by using the present strain (AZ-5512) has a lot of sweetness, as it has a large quantity of amino acids that can be a factor of providing sweetness, and thus, it is possible to obtain a sweet natto product. The details are as described in Fig. 11.

Then, with using Suzumaru soybeans as a raw material, viscosities of the natto produced by using the present strain (AZ-5512) and the natto fermented by using Miyagino bacteria which are used for a typical currently marketed natto product were measured by using TVC-5 VISCOMETER manufactured by Toki Sangyo Co., Ltd.(Japan), and then the viscosities thereof were compared.

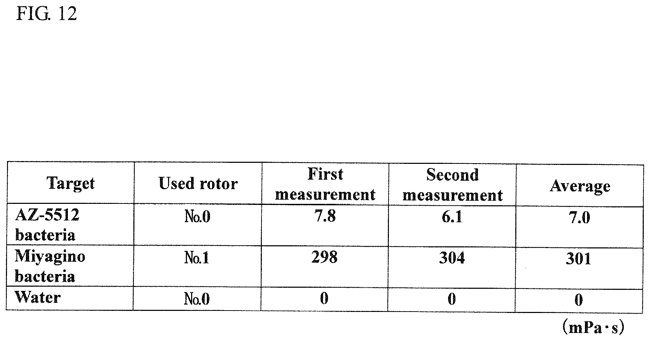

The measurement method was as follows. 150 g of natto for analysis was placed in each Stomacher bag, added with 300 ml of tap water to be dissolved, and then left to stand at room temperature. Every 20 minutes, the natto in the bags was quietly agitated for approximately 30 seconds without forming bubbles in water, and sticky components on the surface of the natto were uniformly eluted in water. At the final step after 60 minutes, the natto in the bags was agitating in the same manner and then filtered through a Japanese bamboo colander to collect 200 ml or more of filtrate in a 500 ml stainless steel beaker. The rotor of a viscometer was installed in a state where the rotor was dipped in the filtrate in the beaker. In the case of the typical natto, absolute viscosity was measured by using the general Rotor No. 1 (50 to 500 mPa.s). In a case where a measurement value was lower than the range of Rotor No. 1, the Rotor No. 0 (0 to 100 mPa·s) of which the measurement range is widened to the lower viscosity side, was used. The results are showned in Fig. 12.

A reference value of viscosity of the applicant company for typical currently marketed product is 220 to 240 and, if the viscosity is less than 140, it is decided that the sticky threads are poor. From the result at this time, an average viscosity of the natto fermented by using Miyagino bacteria is 301 mPa·s and thus sticky threads is considerably preferable. Compared to this, an average viscosity of the natto fermented from the same soybeans as a raw material by using the present strain (AZ-5512) is extremely low, that is, 7.0 mPa·s. This is a remarkable characteristic of the present strain (AZ-5512).

Next, odor components of natto will be described.

Volatile components of the natto produced by using the present strain (AZ-5512) and the natto produced by using the general Bacillus subtilis var. natto were compared. The volatile components of natto were analyzed by capillary gas chromatography and mass spectrometry (GC-MS). The analysis conditions were as follows.

The GC conditions were as follows.
Gas chromatography········ GC 14B type manufactured by Shimadzu Corporation(Japan)
Capillary column ··················· DB-Wax (0.53 mm inner diameter × 30 m length, film thickness of 1 µm, manufactured by Agilent Technologies, Japan)
Carrier gas ··············· Helium: flow rate of 35 cm/sec: implantation temperature of 250°C; column initial temperature: 50°C, increasing rate of temperature of 4°C/min: detector: FID

The MS conditions were as follows.
Instrument ·············· JMS-DX303 manufactured by JEOL Ltd., Japan

| | |
|---|---|
| Temperature of ion source | 200°C |
| Ionization voltage | 70 V |
| Mass range | 35 to 400 amu |
| Scanning period | 1 scan/sec |
| Detection | RI detector |

The method was as follows.

10 g of natto was placed in a 500 ml conical flask. The flask was connected to Tenax-TA column (3 mm diameter × 15 cm length), helium gas was flowed at a flow rate of 10 ml/min for 50 minutes into the conical flask in which the specimen was placed, and then volatile components of the specimen were collected. Upon completion of colleting, Tenax-TA column was connected to the upper end of DB-Wax column, the column outlet was branched in two directions using fused silica tubes by using an X-shaped capillary connector, while makeup gas was added. One of the tubes was guided to the FID for peak detection and the other was guided to the outside of a gas chromatography oven to examine 10 kinds of components similar to natto odor by using a sniffing (sniffing odor by a person) method.

### Regarding Concentration Comparison of Volatile Substance

The same column was connected to a gas chromatograph/mass spectrometer JMS-DX303 manufactured by JEOL Ltd., Japan and analyzed under the same condition so as to perform fixation of odor substances. Moreover, the mass spectrum was measured by scanning in a range of 35 to 400 amu for one second and spectrum obtained by an electron bombardment method was compared with the database stored in a computer (DAWIN) manufactured by JEOL Ltd., Japan to perform fixation of peaks. Further, by comparing a retention index (hereinafter, referred to as RI) with a standard sample, reliable identification was realized.

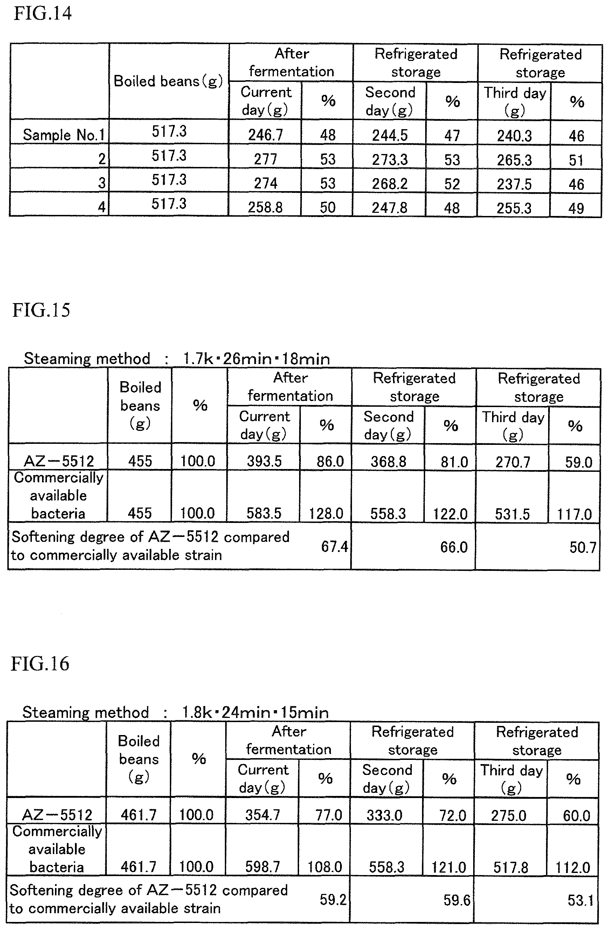

In some of peaks, the molar weight was estimated from CI spectrum obtained by using methane as a reaction gas and thus reliable fixation was realized. The peaks of odor substances obtained by GC were fixed by comparing to RI obtained by GCMS. An area value of peaks obtained by performing the detection by GC using FID was compared to a peak area value of an internal standard material and the results are presented in Fig. 14.

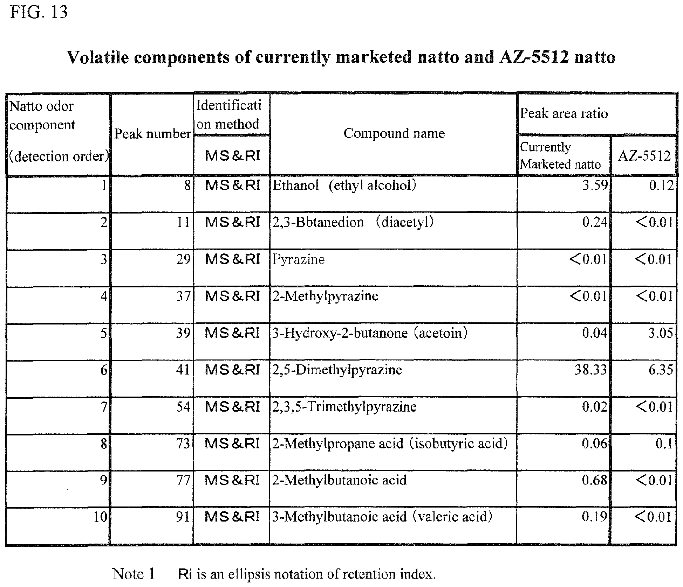

As illustrated in Fig. 13, main components relating to natto odor are the following 10 components. That is,
1. Ethanol
2. Diacetyl
3. Pyrazine
4. 2-Methylpyrazine
5. Acetoin
6. 2,5-Dimethylpyrazine
7. 2,3,5,-Trimethylpyrazine
8. Isobutyric acid
9. Isovaleric acid
10. 2-Methylbutyric acid

Among these, three components of "diacetyl", "isovaleric acid" and "2-methylbutyric acid" are not detected in the natto produced by using the present strain (AZ-5512), and thus the natto is considered to have reduced natto odor.

Next, a hardness test of natto will be described. The hardness test of natto was carried out on the basis of a technology of measuring steam-cooked soybeans and natto according to "Method of Natto Research" edited by Society for Study of Natto. This test was performed as follows. Steam-cooked soybeans and natto were cooled to room temperature while preserving moisture of the steam-cooked soybeans and the natto. Then, the steam-cooked soybeans and the natto were placed on a scale balance one by one and crushed by using the forefinger. The number of grams when they were crushed was used as a hardness. In this test, an average value of 60 grains was calculated.

### 1. Natto Softening Test by Using Micro-Grain Soybeans Produced in USA

(1) Test method
   Production Condition
   (1-1) Steam condition 1.6 k - 26 min - 20 min
   (1-2) Inoculum dose of bacteria: Neat liquid 2.0 × 108
   (1-3) Fermentation condition
      Temperature process: 43°C for 8 hr, 46°C for 5 hr, 43°C for 7 hr, 20°C for 2 hr
      Humidity process: Initial humidity of 80% or higher
   (1-4) Refrigerated storage 5°C
(2) The hardness and reduction rates of hardness of the steam-cooked soybeans and the natto produced by using the present strain (AZ-5512) are showned in Fig. 14.
(3) Result
   (3-1) Although the test was carried out on the same raw materials of soybeans, the hardness of the natto was 50% on the day after fermentation, compared to the steam-cooked soybeans.
   (3-2) Change in hardness was not observed during three days of refrigerated storage.

### 2. Natto Softening Degree Comparison Test of Commercially Available Strain and the Present Strain (AZ-5512) by Using Heterogeneous Soybeans

### (1) Test Method

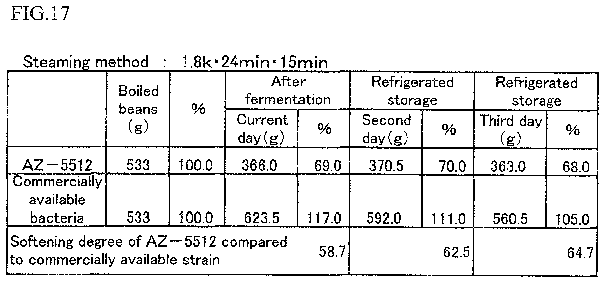

AZ=5512 strains and commercially available bacteria were inoculated on three different kinds of soybeans, respectively, and the comparison test of softening degree was carried out. The commercially available bacteria were Suzumaru (Fig. 15), Zizuka (Fig. 16), and Toyomasari (Fig. 17) .

### (2) Test Result

(2-1) Compared to the hardness of the steam-cooked soybeans after completion of fermentation, the hardness in the case of the present strain (AZ-5512) was decreased by 80 to 70%. However, in the case of the commercially available bacteria, the hardness was increased by 110 to 130%. Therefore, the difference in hardness was approximately 60%.
(2-2) In the case of the present strain (AZ-5512), the soybeans were softened after completion of fermentation. In the case of commercially available bacteria, softening tendency was shown after three days of refrigerated storage but the hardness did not reach to the hardness of boiled soybeans.
(2-3) In the case of the present strain (AZ-5512), the soybeans were softened after completion of fermentation regardless of breed of raw materials of soybeans.

### INDUSTRIAL APPLICABILITY

According to the present invention, it is possible to obtain soft natto having a small number of viscous substances, little natto odor, and a lot of sweetness. Accordingly, new development in application field of natto can be expected.

For example, the natto of the present invention can be used in oil frying, natto tempura (natto fritter), fry, or the like. In the case of conventional natto, occurrence of ammonia odor and degradation of tempura oil are raised. However, by using the natto of the present invention, it is possible to reduce such degradation as much as possible.

Specifically, the natto of the present invention can be used in snacks, desserts, sweets, and the like as food for women's beauty. The natto of the present invention has favorable appearance. In particular, the black bean natto is merit in color and has sweet flavor and sweet taste, so that it can be used as a topping for a cake, or the like. In addition, the black bean natto is put into a cake to be baked and thus nutrient enhancement can be achieved.

While nourishment and food functionality of natto can be expected, some people cannot eat natto because of resistance to natto having sticky and slimy texture and natto odor, and particularly, foreigners have strong resistance to natto. However, the natto of the present invention can be provided as raw diet to these people.

Moreover, the natto of the present invention may be applicable to people who need soft natto because of incomplete mastication, such as babies or people who live with medical care.

## Claims

1. Bacillus subtilis AZ-5512 strain (FERM P-22135).

2. Natto produced by using the Bacillus subtilis AZ-5512 strain (FERM P-22135).

## Patentansprüche

1. Bacillus subtilis AZ-5512-Stamm (FERM P-22135).

2. Natto, welches unter Verwendung des Bacillus subtilis AZ-5512-Stamms (FERM P-22135) hergestellt wird.

## Revendications

1. Souche de Bacillus subtilis AZ-5512 (FERM P-22135).

2. Natto produit en utilisant la souche de Bacillus subtilis AZ-5512 (FERM P-22135).
